Europäisches Patentamt

European Patent Office

Office européen des brevets

(11) Publication number: **0 200 374 B1**

(12) **EUROPEAN PATENT SPECIFICATION**

(45) Date of publication of the patent specification: 09.08.89

(51) Int. Cl.⁴: **A61B 6/03, A61B 6/04**

(21) Application number: 86302428.7

(22) Date of filing: 02.04.86

(54) **Shadowgraph imaging.**

(30) Priority: 12.04.85 US 722672

(43) Date of publication of application: 10.12.86 Bulletin 86/45

(45) Publication of the grant of the patent: 09.08.89 Bulletin 89/32

(84) Designated Contracting States: DE FR GB NL

(56) References cited:
EP-A- 0 041 752
EP-A- 0 041 752
EP-A- 0 077 447
EP-A- 0 117 524
DE-A- 3 018 541
DE-A- 3 227 625
US-A- 4 179 100

OPTICAL ENGINEERING, vol. 17, no. 6, November-December 1978, pages 652-657; R.A. KRUGER et al.: "A digital video image processor for real-time X-ray subtraction imaging"

(73) Proprietor: PICKER INTERNATIONAL, INC., 595 Miner Road, Highland Heights Ohio 44143(US)

(72) Inventor: DeMeester, Gordon D., 1613 Dennis Drive, Wickliffe, Ohio 44092(US)
Inventor: Mattson, Rodney A., 6532 Melshore Drive, Mentor, Ohio 44060(US)

(74) Representative: Pope, Michael Bertram Wingate, Central Patent Department Wembley Office The General Electric Company, p.l.c. Hirst Research Centre East Lane, Wembley Middlesex HA9 7PP(GB)

## Description

This invention relates to methods and apparatus for use in obtaining shadowgraph images and more especially to such methods and apparatus for use in locating a tomographic plane to be scanned using a computed tomography (CT) apparatus.

In computed tomography (CT), a subject is irradiated from a number of directions and the intensity of radiation passing through the subject is detected. This procedure provides information sufficient to generate a cross-sectional mapping of attenuation within the subject. The procedure for generating this mapping is known within the computed tomography art as filtered back projection. Various methods of reconstruction imaging have been developed as the computer tomography art has matured. In most all, if not all, commercial CT scanners presently known to applicants, x-ray attenuation data are fourier transformed, filtered, and then retransformed prior to back projection.

Computed tomography scanners have evolved in stages. An initial scanner used a single x-ray source and single detector which traversed back and forth in unison obtaining intensity information for use in the back projection process. Second and third generation scanners employed detector arrays rather than individual detectors. In second generation scanners these arrays traversed and orbited while in third generation they simply orbit with the x-ray source. A fourth generation scanner employs a circular array of detectors circumscribing a subject region of interest. The x-ray source orbits with respect to this circular detector array to irradiate the subject from a plurality of positions.

One example of a fourth generation medical computed tomography scanner is marketed under the designation Synerview 1200 by Picker International, Inc. of Cleveland, Ohio. In this scanner, the detector array includes 1200 closely spaced detectors forming a circle whose diameter is greater than the diameter of the circular path followed by the x-ray tube as it orbits the patient. Stated another way, x-radiation from the tube travels a shorter distance between tube and patient than between the patient and detector array after traversing the patient.

One requirement of computed tomography scanners is a procedure for accurately positioning the subject prior to conducting a computed tomography scan. For example, if a diagnosing physician is interested in viewing the internal structure of a particular organ, it is inefficient and undesirable to scan the entire body. Instead, only a region of interest including the organ or other body part of concern is scanned.

To accomplish this positioning the x-ray source can be locked in a stationary position relative to the detector array and the subject moved past the array as the source irradiates the subject. X-ray intensity data from a group of detectors is sensed sequentially at each of a series of data collection positions. At each data collection position a new row of intensity information is gathered. After the subject has moved past the detector array an image similar to a conventional digital x-ray shadowgraph image is created. Such an image does not represent a cross-sectional image but instead, depicts the transmissivity of the subject along paths from the fixed x-ray source to individual elements of the detector array. While not providing the resolution and/or information of a computed tomography scan, such a localization scan can be used to locate structure within the subject in anticipation of positioning that subject for a complete CT scan.

Such a localization scan is described in EP-A 10 041 752. In EP-A 10 041 752 there is further described a method comprising carrying out localization scans to obtain two sets of shadowgraph image data, which differ only in respect of an image determining parameter and substracting one from the other. The difference in an image determining parameter may, for example, be due to the injection of a contrast medium into the blood flow in a subject so that the difference image reveals the location of blood vessels in the subject. EP-A 10 041 752 also describes the formation of a pair of shadowgraph images to provide a stereoscopic view of a subject, and the formation of a series of shadowgraph images for kinematographic presentation.

It is an object of the present invention to provide a method and apparatus for obtaining a shadowgraph image of improved resolution for localization purposes prior to a complete CT scan.

Accordingly the invention provides a method of obtaining shadowgraph image data relating to a subject wherein: radiation from a radiation source is directed through the subject to an array of radiation detectors; and data is obtained representing the radiation reaching said array through said subject as said subject is moved relative to said source and said array orthogonally to the radiation path; data being obtained during two corresponding movements of said subject relative to said source and said array; and said data for the two movements being combined to produce said image data; characterised in that the said radiation is directed along trajectories through said subject during one said movement which are different from the trajectories during the other said movement; and said data for the two movements are combined in an array by interleaving said data so as to provide image data of greater resolution than that given by the data for either one movement alone.

The invention also provides an apparatus for producing a shadowgraph image of a subject comprising: a radiation source; an array of radiation detectors; means positioning said source and array so as to direct radiation through said subject onto said array; means for moving said subject orthogonally to the radiation path and relative to said source and said array; means for producing signals representing the radiation reaching said array from the source through said subject as said subject is moved relative to said source and said array; and means for utilising said signals to construct said image; said means

EP 0 200 374 B1

for moving comprising means for carrying out two corresponding movements of said subject relative to said source and said array; and said means for utilising combining the said signals produced during both said movements; characterised in that said means for positioning includes means for moving said source relative to said subject and said array between sais movements so that the said radiation is directed along trajectories through said subject during one said movement which are different from the trajectories during the other said movement; and said means for utilising combines the signals produced during said two movements in an array by interleaving said data so as to construct an image of greater resolution than that given by the signals produced during either one said movement alone.

It will be appreciated that in a method and apparatus according to the invention said source and detector array may be the source and detector array of a CT scaner apparatus.

One method and apparatus in accordance with the invention will now be described by way of example with reference to the accompanying drawing in which: –

Figure 1 is a perspective view of a medical computed tomography scanner having a motorized couch for moving a patient relative to a scanner x-ray tube and stationary detector array;

Figure 2 is a side elevation view of the patient couch having a motor for moving the patient longitudinally into and out of a scanner patient aperture;

Figure 3 is an end elevation view of the Figure 2 couch;

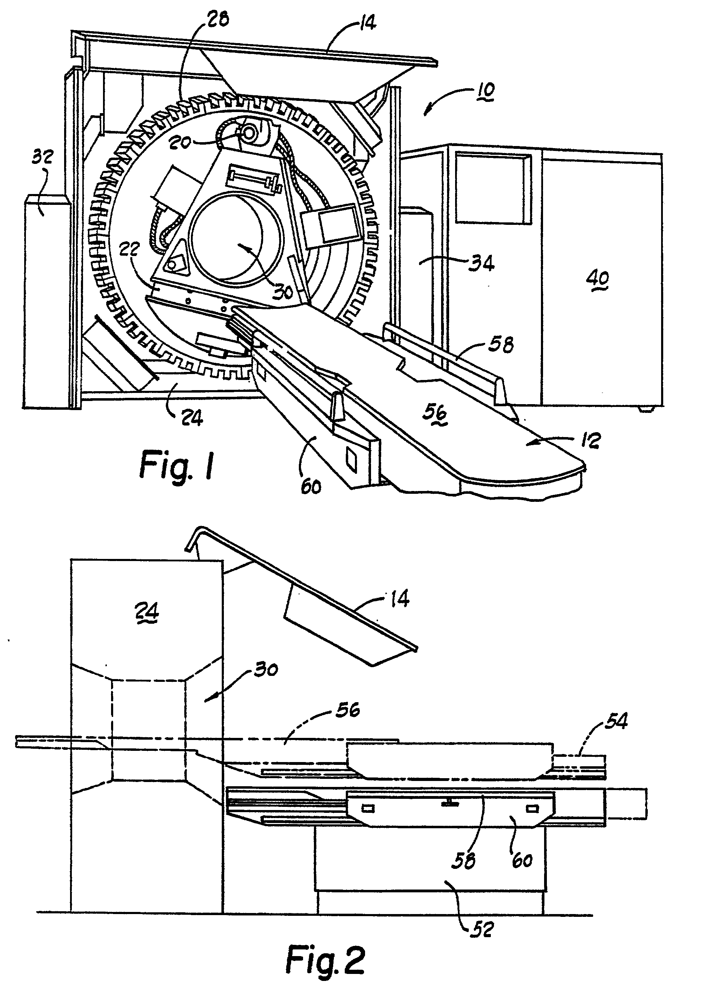

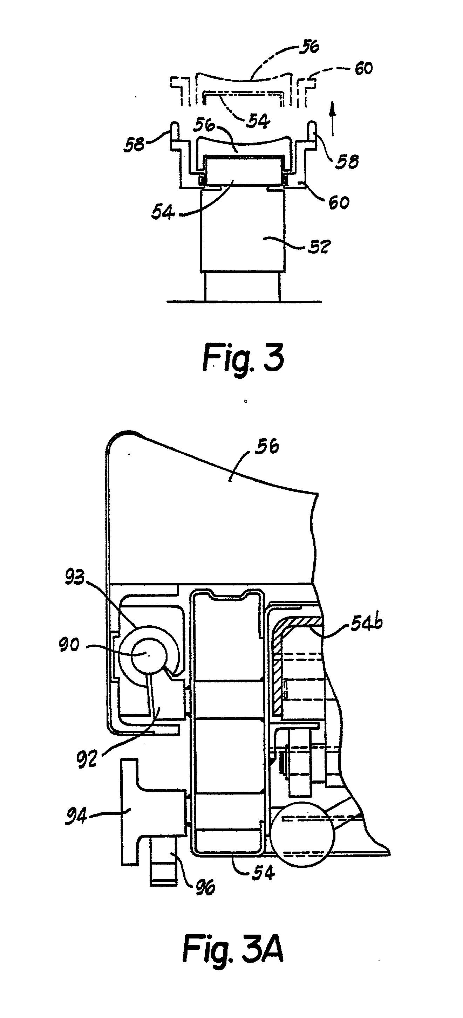

Figure 3 is an end elevation view of the Figure 2 couch;

Figure 3A is an enlarged partially sectioned, view of the couch top and its support;

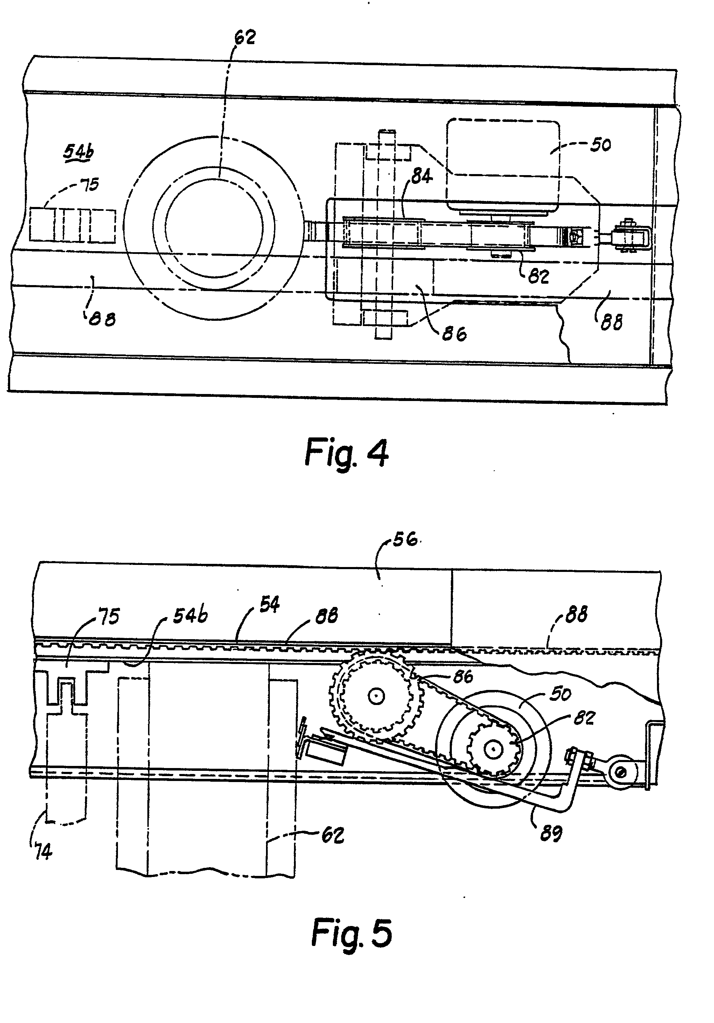

Figure 4 is a top plan view of the couch frame showing a drive for moving a patient support into and out of a scanner aperture;

Figure 5 is a side elevation view, partly in section, of the Figure 4 drive;

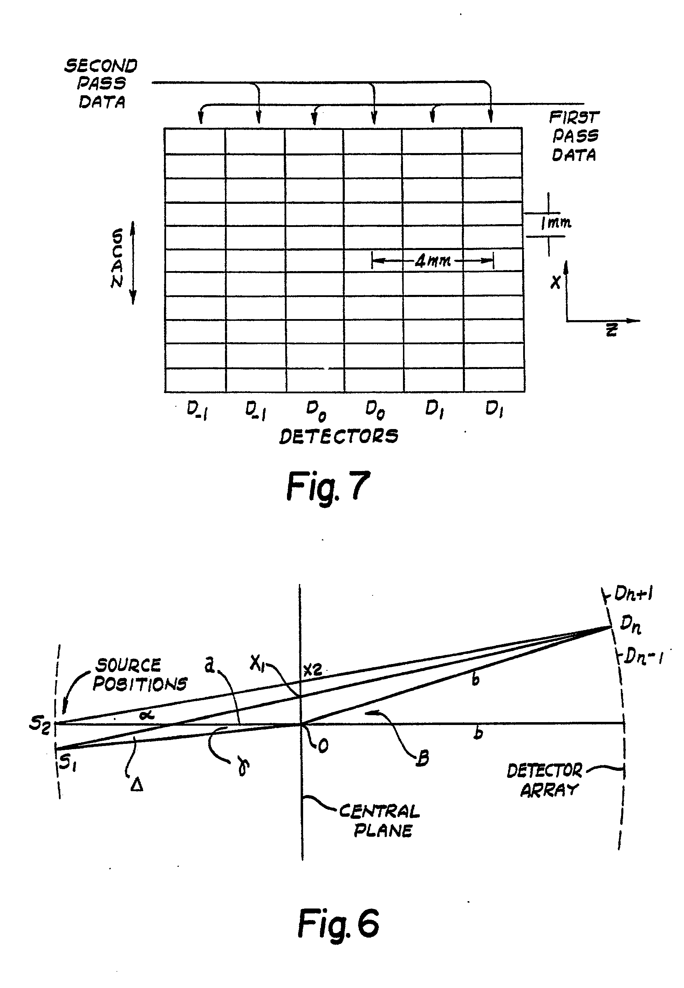

Figure 6 is a schematic showing a relationship between the scanner x-ray tube and a portion of the scanner detector array to illustrate an interleaving of shadowgraph image data; and

Figure 7 is a mapping of interleaved intensity data showing increased image resolution through practice of the invention;

Turning now to the drawings, and in particular Figure 1, a computed tomography scanner 10 and a patient couch 12 are illustrated. The scanner 10 is illustrated with a front panel 14 pivoted away from its in use position to show the internal components of the scanner 10. The scanner 10 includes a rotatable x-ray source 20 mounted on a rotatable frame 22. The frame 22 is journalled for rotation within a gantry 24. Coupled to and supported by the gantry 24 are a plurality of x-radiation detectors forming a detector array 28. During operation radiation from the source 20 passes across a patient aperture 30 in a plane 32 to the detector array 28.

During computed tomography scanning, the x-ray source 20 is rotated by a motor (not shown) in a circular orbit around a patient aperture 30. A generally planar and spread beam of radiation from the source 20 impinges upon a group of detectors forming a part of the detector array 28. As the source rotates, different detectors are irradiated and intensity information obtained from these detectors. Output signals from the detector array 28 are processed by components that convert the radiation into visible light, to an analog electric signal, to a frequency, and then to digital attenuation values. A computer 40 stores this data derived from these values and uses it for reconstruction processing. As seen most clearly in Figures 2 and 3, the patient couch 12 includes a base 52 supporting a movable frame 54 which supports a top 56. The top 56 and frame 54 are bounded on either side by retractable handles 58 and an arm rest 60. To position a patient in a patient examination position in the patient aperture for computed tomoggraphy scanning, a frame lift motor (not shown) raises the frame 54, top 56 and handles 58 from the position shown in solid in Figure 2 to the position shown in phantom. In this raised position, the patient can be moved into and out of the patient aperture 30.

A longitudinal drive mechanism for the couch frame 54 and top 56 is seen in Figure 5. One of two pedestal supports 62, is shown supporting the frame 54. This support 62 is coupled to a second pedestal (not shown) by a shaft and rack and pinion drive so that the two pedestal supports move in unison as the frame is raised and lowered. A motor driven screw jack 74 is coupled to a mounting block 75 which engages a bottom surface 54b of the frame 54.

Longitudinal movement of the top 56 with respect to the couch frame 54 is provided by a D.C. motor 50 mounted within the frame 54. A drive pulley 82 mounted to a motor drive shaft is rotated in either of two directions. A pulley 84 (Figure 4) mounted to a shaft 85 journaled in the frame 54 is connected to the drive pulley 82 by a belt 87 and rotates in response to energization of the motor 50. A gear 86 mounted on the shaft 85 engages an elongated gear belt 88 coupled to the movable top 56 so that rotation of the gear 86 exerts a force on the moveable top 56. Controlled energization of the motor 50 drives the gear belt and attached top 56 into and out of the aperture 30.

The top 56 and frame 54 coact along two bearing rails 90 (only one of which is seen in Figure 3A). Each rail is mounted on a shaft support block 92 (one of which is shown in Figure 3A). The rails 90 each engage bearings 93 running the length of the moveable top 56. Directly beneath the rail 90 (Figure 3A) are side rails 94 which support the arm rests 60 (Figure 3).

As has been indicated the patient couch 12 is motorized so that a patient placed on the couch can be

3

moved into the scanner aperture 30. The patient is moved into the aperture 30 so that radiation from the tube 20 passes through the patient cross-section 32 (Figure 2).

In preparation for cross-sectional scanning, a shadowgraph patient image is obtained. The x-ray tube 20 is fixed with respect to the gantry 24 and a beam of x-radiation is directed through the patient to a segment of adjacent detectors of the detector array 28. The x-ray tube 20 remains energized as the motor 50 continuously moves the patient axially of the aperutre 30 and through the beam of radiation. X-radiation data from the energized x-ray detector segment is sensed and stored for the time period in which the patient is driven with respect to the scanner through a first longitudinal scan. During this first scan, movement of the top 56 is co-ordinated with radiation attentuation sensing so the data stored in the computer is organized in a rectangular array of pixels of attenuation data. In one embodiment of the invention for use with a 600 detector scanner, the radiation output from 128 adjacent detectors is sampled every millimeter at 128 different patient/scanner positons to obtain a 128 by 128 pixel shadowgraph data set.

After the first longitudinal scan is conducted, the source 20 is orbitally indexed a small amount to provide a second shadowgraph data set. In a 600 detector scanner, where the spacing between detectors is 0.6 degrees, the source is moved .3 degrees from its original position. The motor 50 is then energized to reverse the longitudinal scanning direction. The top 56 then moves in an opposite direction as a second set of shadowgraph data is obtained. The pixel data for each of the two shadowgraph images is then interleaved by the computer 40 to obtain a resultant shadowgraph of 256 by 128 pixels having twice the data set resolution of either individual longitudinal scan.

A grid like mapping (Figure 7) of data from the 128 detectors is created. Along the longitudinal direction the center-to-center spacing between adjacent data regions is 1 mm. Without multiple scans the detector spacing is approximately 4 mm but with the interleaving of data from two scans with the source orbited between scans the separation between data regions is about 2 mm.

Turning now to Figure 6, a geometric examination of shadowgraph data interleaving is presented. In Figure 6, two source positions S1 and S2 are seen in relation to a detector array having representative detectors $D_n$, $D_{n+1}$, and $D_{n-1}$. The angle $\gamma$ represents the angle by which the scanning frame is rotated between longitudinal scans. Depicted in a central scanning plane are two points $X_1$, $X_2$. The following two equations represent the distance of these points $X_1$, $X_2$ from the origin O as a function of the Figure 6 parameters.

$$X_2 = a \tan \left[ \arcsin \left( \frac{b \sin \beta}{\sqrt{a^2 + b^2 + 2ab \cos \beta}} \right) \right]$$

$$X_1 = \frac{a \sin \Delta}{\cos (\gamma + \Delta)} \quad , \text{where}$$

$$\Delta = \arcsin \left[ \frac{b \sin (\beta - \gamma)}{\sqrt{a^2 + b^2 + 2ab \cos (\beta - \gamma)}} \right]$$

These equations are derived from the law of sines and co-sines for the geometry of the Figure 6 representation. A tabulation of these values for $X_1$, $X_2$ for representation detectors of a 600 detector scanner is summarized in Table I. That table shows the distance along the central plane from the origin to the locations $X_1$ and $X_2$ for various adjacent detectors for two source positions .3° degrees apart. By way of example, the designation $X_2$ (0,3) means the distance from $X_2$ to the origin in the central plane for the ray passing from the source at position $S_2$ to the third detector in the detector array.

4

Table I

| $X_2 = X_2(0, n)$ | | | $X_1 = X_1(.3, n)$ | | |
|---|---|---|---|---|---|
| $X_2(0,0)$ | = | 1.91 mm | $X_1(.3,0)$ | = | 0.0 mm |
| $X_2(0,1)$ | = | 5.74 mm | $X_1(.3,1)$ | = | 3.82 mm |
| $X_2(0,2)$ | = | 9.56 mm | $X_1(.3,2)$ | = | 7.65 mm |
| $X_2(0,3)$ | = | 13.38 mm | | | |
| $X_2(0,31)$ | = | 122.02 mm | | | |
| $X_2(0,32)$ | = | 125.99 mm | $X_1(.3,32)$ | = | 124.13 mm |
| $X_2(0,63)$ | = | 256.45 mm | | | |
| $X_2(0,64)$ | = | 260.96 mm | $X_1(.3,64)$ | = | 259.53 mm |

## Claims

1. A method of obtaining shadowgraph image data relating to a subject wherein: radiation from a radiation source (20) is directed through the subject to an array of radiation detectors (28); and data is obtained representing the radiation reaching said array (28), through said subject as said subject is moved orthogonally to the radiation path and relative to said source (20) and said array (28); data being obtained during two corresponding movements of said subject relative to said source (20) and said array (25); and said data for the two movements being combined to produce said image data; characterised in that the said radiation is directed along trajectories through said subject during one said movement which are different from the trajectories during the other said movement; and said data for the two movements are combined in an array by interleaving said data so as to provide image data of greater resolution than that given by the data for either one movement alone.

2. A method according to claim 1 wherein said two movements are movements in opposite directions.

3. A method according to claim 1 or claim 2 wherein said different trajectories are obtained by positioning said source (20) at a different angular position (S1, S2) about the direction of said movements during each of said two movements.

4. A method according to claim 3 wherein said array (28) is a circular array and said different angular positions (S1, S2) lie on a circula path having the same centre as said array (28).

5. A method according to claim 4 wherein the angular position (S1, S2) of said source (20) during said two movements differ by an angle ($\gamma$) approximately one half of the angular spacing between adjacent detectors in said array.

6. A method according to any one of the preceding claims wherein said source (20) and said array (28) are positioned around an aperture (30) and said movements are in a direction axial of said aperture (30).

7. A method according to any one of the preceding claims wherein during each said movement data is obtained for each of a number of discrete positions of said subject relative to said source (20) and said array (28).

8. A method according to any one of the preceding claims wherein said movements are effected by moving said subject whilst said array (28) and said source (20) remain fixed in position.

9. A method according to any one of the preceding claims wherein said source (20) is an X-ray source.

10. A method according to any one of the preceding claims wherein said method is carried out using a computed tomography scanner apparatus (10), said source (20) and said array (28) being a sourche and array used to produce computed tomography scans in use of said apparatus (10).

11. An apparatus for producing a shadowgraph image of a subject comprising: a radiation source (20); an array of radiation detectors (28); means (22, 24) positioning said source (20) and array (28) so as to direct radiation through said subject onto said array (28); means (12, 24, 50 to 62, 82 to 94) for moving said subject relative to said source (20) and said array (28) orthogonally to the radiation path; means (40) for producing signals representing the radiation reaching said array (28) from the source (20) through said subject as said subject is moved relative to said source (20) and said array (28); and means (40) for utilising said signals to construct said image; said means (12, 24, 50 to 62, 82 to 94) for moving comprising means for carrying out two corresponding movements of said subject relative to said source (20) and said array (28); and said means (40) for utilising combining the said signals produced during both said movements; characterised in that said means (22, 24) for positioning includes means (22) for moving said source (20) relative to said subject and said array (28) between said movements so that the said radiation is directed along trajectories through said subject during one said movement which are different from the trajectories during the other said movement; and said means (40) for utilising combines the signals produced during said two movements in an array by interleaving said data so as to construct an image of greater resolution than that given by the signals produced during either one said movement alone.

12. An apparatus according to claim 11 wherein said means (22) for moving said source (20) comprises means (22) for moving said source (20) to different positions (S1, S2) along a circular path around the direction of said movements.

13. An apparatus according to claim 12 wherein said array (28) is a circular array having the same centre as said circular path.

14. An apparatus according to any one of claims 11 to 13 wherein said source (20) and said array (28) are positioned around a circular aperture (30) and said movements are in a direction axial of said aperture (30).

15. An apparatus according to any one of claims 11 to 14 wherein said source (20) and said array (28) are radiation source and detector array of a computed tomography scanner apparatus (10).

**Patentansprüche**

1. Verfahren zur Gewinnung von Übersichtsaufnahme-Bilddaten bezüglich eines Objekts, bei dem die Strahlung einer Strahlungsquelle (20) durch das Objekt hindurch auf eine Reihe von Strahlungsdetektoren (28) gerichtet wird und die die Detektorreihe (28) durch das Objekt hindurch erreichende Strahlung darstellende Daten gewonnen werden, während das Objekt senkrecht zur Strahlungsbahn und relativ zur Strahlungsquelle (20) und der Detektorreihe (28) bewegt wird, wobei Daten während zweier entsprechender Bewegungen des Objekts relativ zur Strahlungsquelle (20) und der Detektorreihe (28) gewonnen werden und die Daten für die beiden Bewegungen kombiniert werden, um die Bilddaten zu erzeugen, dadurch gekennzeichnet, daß während der einen Bewegung die Strahlung durch das Objekt hindurch längs Bahnen gerichtet wird, die von den Strahlungs-Bahnen während der anderen Bewegung abweichen, und daß die Daten für die beiden Bewegungen durch eine derartige Verschachtelung der Daten in einer Reihe kombiniert werden, daß Bilddaten mit größerer Auflösung als derjenigen erzeugt werden, die sich durch die Daten bei einer der beiden Bewegungen allein ergeben.

2. Verfahren nach Anspruch 1, bei dem die beiden Bewegungen entgegengesetzt gerichtet sind.

3. Verfahren nach Anspruch 1 oder 2, bei dem die abweichenden Bahnen dadurch bewirkt werden, daß die Strahlungsquelle (20) während jeder der beiden Bewegungen in verschiedenen Winkellagen (S1, S2) um die Richtung der Bewegungen angeordnet wird.

4. Verfahren nach Anspruch 3, bei dem die Detektorreihe (28) kreisförmig ist und die verschiedenen Winkellagen (S1, S2) auf einer Kreisbahn mit dem gleichen Mittelpunkt wie die Detektorreihe (28) liegen.

5. Verfahren nach Anspruch 4, bei dem die Winkellagen (S1, S2) der Strahlunsquelle (20) während der beiden Bewegungen um einen Winkel ($\gamma$) voneinander abweichen, der etwa gleich dem halben Winkelabstand benachbarter Detektoren in der Detektorreihe ist.

6. Verfahren nach einem der vorstehenden Ansprüche, bei dem die Strahlungsquelle (20) und die Detektorreihe (28) um eine Öffnung (30) herum angeordnet und die Bewegungen axial zur Öffnung (30) gerichtet sind.

7. Verfahren nach einem der vorstehenden Ansprüche, bei dem während jeder der Bewegungen Daten in jeder von einer Anzahl einzelner Positionen des Objekts relativ zur Strahlungsquelle (20) und Detektorreihe (28) gewonnen werden.

8. Verfahren nach einem der vorstehenden Ansprüche, bei dem die Bewegungen dadurch bewirkt werden, daß das Objekt bewegt wird, während die Detektorreihe (28) und die Strahlungsquelle (20) eine feste Lage beibehalten.

9. Verfahren nach einem der vorstehenden Ansprüche, bei dem die Strahlungsquelle (20) eine Röntgenstrahlungsquelle ist.

10. Verfahren nach einem der vorstehenden Ansprüche, bei dem das Verfahren mittels eines Computertomographie-Abtastgerätes (10) ausgeführt wird, wobei die Strahlungsquelle (20) und die Detektorreihe (28) eine Strahlungsquelle und Detektorreihe sind, wie sie zur Erzeugung von Computertomographieabtastungen bei Verwendung des erwähnten Gerätes (10) verwendet werden.

11. Vorrichtung zum Erzeugen eines Übersichtsaufnahmebildes eines Objekts, die aufweist: eine Strahlungsquelle (20); eine Reihe von Strahlungsdetektoren (28); eine die Strahlungsquelle (20) und die Detektorreihe (28) so positionierende Einrichtung (22, 24), daß die Strahlung durch das Objekt hindurch auf die Detektorreihe (28) gerichtet ist; eine Einrichtung (12, 24, 50–62, 82–94) zum Bewegen des Objekts relativ zur Strahlungsquelle (20) und zur Detektorreihe (28) senkrecht zur Strahlungsbahn; eine Einrichtung (40) zum Erzeugen von Signalen, die die Strahlung darstellen, die die Detektorreihe (28) von der Strahlungsquelle (20) durch das Objekt hindurch erreichen, während das Objekt relativ zur Strahlungsquelle (20) und zur Detektorreihe (28) bewegt wird, und eine Einrichtung (40) zum Auswerten der Signale, um das Bild aufzubauen; wobei die Bewegungseinrichtung (12, 24, 50–62, 82–94) eine Einrichtung zum Ausführen zweier korrespondierender Bewegungen des Objekts relativ zur Strahlungsquelle (20) und zur Detektorreihe (28) aufweist und die Auswertungseinrichtung (40) die während beider Bewegungen erzeugten Signale kombiniert, dadurch gekennzeichnet, daß die Positioniereinrichtung (22, 24) eine Einrichtung (22) zum Bewegen der Strahlungsquelle (20) relativ zum Objekt und der Detektorreihe (28) zwischen den Bewegungen aufweist, so daß die Strahlung, während der einen Bewegung, längs das Objekt durchsetzender Bahnen gerichtet ist, die von den Bahnen während der anderen Bewegung verschieden sind, und die Auswertungseinrichtung (40) die während der beiden Bewegungen in einer Reihe erzeugten Signale durch eine derartige Verschachtelung der Daten kombiniert, daß ein Bild mit größerer Auflösung als dasjenige aufgebaut wird, das sich durch die Signale ergibt, die während einer der beiden Bewegungen allein erzeugt werden.

12. Vorrichtung nach Anspruch 11, bei der die Einrichtung (22) zum Bewegen der Strahlungsquelle (20) eine Einrichtung (22) zum Bewegen der Strahlungsquelle (20) in verschiedene Positionen (S1, S2) längs einer kreisförmigen Bahn um die Richtung der Bewegungen herum aufweist.

13. Vorrichtung nach Anspruch 12, bei der die Detektorreihe (28) kreisförmig ist und den gleichen Mittelpunkt wie die kreisförmige Bahn aufweist.

14. Vorrichtung nach einem der Ansprüche 11 bis 13, bei der die Strahlungsquelle (20) und die Detektorreihe (28) um eine kreisförmige Öffnung (30) herum angeordnet und die Bewegungen axial zur Öffnung (30) gerichtet sind.

15. Vorrichtung nach einem der Ansprüche 11 bis 14, bei der die Strahlungsquelle (20) und die Detektorreihe (28) eine Strahlungsquelle und Detektorreihe eines Computertomographie-Abtastgerätes (10) sind.

**Revendications**

1. Procédé d'obtention de données d'image par ombre relatives à un sujet, dans lequel le rayonnement d'une source (20) est dirigé à travers le sujet vers un arrangement de détecteurs (28) du rayonnement, et des données sont obtenues de manière qu'elles représentent le rayonnement atteignant l'arrangement (28) après déplacement du sujet lorsque le sujet est déplacé perpendiculairement à la trajectoire du rayonnement et par rapport à la source (20) et à l'arrangement (28), des données étant obtenues pendant deux déplacements correspondants du sujet par rapport à la source (20) et à l'arrangement (28), et des données des deux déplacements étant combinées afin qu'elles donnent des données d'image, caractérisé en ce que le rayonnement est dirigé, pendant le premier déplacement, suivant des trajectoires qui traversent le sujet et qui sont différentes des trajectoires utilisées pendant l'autre déplacement, et les données des deux déplacements sont combinées dans un tableau par entrelacement des données de manière qu'elles forment des données d'image de résolution supérieure à celle qui est donnée par les données de l'un ou l'autre déplacement seul.

2. Procédé selon la revendication 1, dans lequel les deux déplacements sont des déplacements de sens opposés.

3. Procédé selon la revendication 1 ou 2, ans lequel les trajectoires différentes sont obtenues par disposition de la source (20) à des positions angulaires différentes (S1, S2) autour de la direction des déplacements dans chacun des deux déplacements.

4. Procédé selon la revendication 3, dans lequel l'arrangement (28) est un arrangement circulaire et les différentes positions angulaires (S1, S2) se trouvent sur un trajet circulaire ayant le même centre que l'arrangement (28).

5. Procédé selon la revendication 4, dans lequel les positions angulaires (S1, S2) de la source (20) pendant les deux déplacements diffèrent d'un angle (γ) à peu près égal à la moitié de la distance angulaire séparant les détecteurs adjacents de l'arrangement.

6. Procédé selon l'une quelconque des revendications précédentes, dans lequel la source (20) et l'arrangement (28) sont disposés autour d'une ouverture (30) et lesdits déplacements s'effectuent dans la direction de l'axe de l'ouverture (30).

7. Procédé selon l'une quelconque des revendications précédentes, dans lequel, pendant chaque déplacement, des données sont obtenues pour chacune d'un certain nombre de position séparées du sujet par rapport à la source (20) et à l'arrangement (28).

8. Procédé selon l'une quelconque des revendications précédentes, dans lequel les déplacements sont réalisés par déplacement du sujet pendant que l'arrangement (28) et la source (20) gardent des positions fixes.

9. Procédé selon l'une quelconque des revendications précédentes, dans lequel la source (20) est une source radiographique.

10. Procédé selon l'une quelconque des revendications précédentes, dans lequel le procédé est mis en œuvre à l'aide d'un appareil tacographique (10), la source (20) et l'arrangement (28) étant constitués par une source et un arrangement utilisés pour l'exécution d'analyse tacographique pendant l'utilisation de l'appareil (10).

11. Appareil de formation d'une image par ombre d'un sujet, comprenant une source (20) d'un rayonnement, un arrangement de détecteurs (28) du rayonnement, un dispositif (22, 24) de positionnement de la source (20) et de l'arrangement (28) de manière que le rayonnement soit dirigé à travers le sujet sur l'arrangement (28), un dispositif (12, 24, 50 à 62, 82 à 94) destiné à déplacer le sujet par rapport à la source (20) et à l'arrangement (28) orthogonalement par rapport à la trajectoire du rayonnement, un dispositif (40) destiné à former des signaux représentant le rayonnement atteignant l'arrangement (28) à partir de la source (20) et traversant le sujet lorsque celui-ci se déplace par rapport à la source (20) et à l'arrangement (28), et un dispositif (40) destiné à utiliser les signaux pour construire l'image, le dispositif (12, 24, 50 à 62, 82 à 94) destiné à déplacer comprenant un dispositif destiné à assurer deux déplacements correspondants du sujet par rapport à la source (20) et à l'arrangement (28), et le dispositif (40) destiné à utiliser assure la combinaison des signaux produits pendant les deux déplacements, caractérisé en ce que le dispositif (22, 24) de positionnement comporte un dispositif (22) destiné à déplacer la source (20) par rapport au sujet et à l'arrangement (28) entre les déplacements afin que le rayonnement soit dirigé, pendant le premier déplacement, suivant les trajectoires traversant le sujet qui sont différentes des trajectoires obtenues pendant l'autre déplacement, et le dispositif (40) destiné à utiliser les signaux combine les signaux produits pendant les deux déplacements dans un tableau par entrelacement des données de manière qu'ils construisent une image de résolution supérieure à celle qui est donnée par les signaux produits pendant l'un ou l'autre des déplacements seulement.

12. Appareil selon la revendication 11, dans lequel le dispositif (22) destiné à déplacer la source (20) comporte un dispositif (22) de déplacement de la source (20) vers des positions différentes (S1, S2) suivant un trajet circulaire autour de la direction des déplacements.

13. Appareil selon la revendication 12, dans lequel l'arrangement (28) est un arrangement circulaire ayant le même centre que le trajet circulaire.

14. Appareil selon l'une quelconque des revendications 11 à 13, dans lequel la source (20) et l'arrangement (28) sont disposés autour de l'ouverture circulaire (30), et les déplacements s'effectuent dans la direction de l'axe de l'ouverture (30).

15. Appareil selon l'une quelconque des revendications 11 à 14, dans lequel la source (20) et l'arrangement (28) sont une source d'un rayonnement et un arrangement de détecteurs d'un appareil de tacographie (10).

*Fig. 1*

*Fig.2*

EP 0 200 374 B1

Fig. 3

Fig. 3A

**Fig. 4**

**Fig. 5**

Fig. 7

Fig. 6